# EUROPEAN PATENT APPLICATION

(11) **EP 3 909 640 A1**
(43) Date of publication of application: **17.11.2021**
(21) Application number: 20382387.7
(22) Date of filing: 12.05.2020
(51) Int. Cl.: A61N 1/05, A61N 1/372, G16H 50/50

(54) **MEDICAL DEVICE FOR SPINAL CORD STIMULATION**

(71) Applicant: Surgicen SLU, 46230 Alginet, Valencia (ES); García Vitoria, Carles, 46003 Valencia (ES)
(72) Inventor: García Vitoria, Carles, 46003 Valencia (ES); Durá Cantero, José Luis, 46014 Valencia (ES)
(74) Representative: Hoffmann Eitle

(57) **Abstract**

Medical device for spinal cord stimulation. The present invention refers to a medical device for spinal cord stimulation which comprises: a) An electrode, consisting essentially of a plurality of poles, suitable for being inserted inside the intradural space through the dura mater via needle-puncture of the skin; and b) an external unit, connected to the electrode, suitable for generating electric current and reading the impedance.

## Description

### FIELD OF THE INVENTION

The present invention refers to the medical field. Specifically, the present invention refers to a medical device for spinal cord stimulation (SCS) which comprises: a) An electrode (1), consisting essentially of a plurality of poles (2), suitable for being inserted inside the intradural space (3) through the dura mater (4) via needle-puncture (5) of the skin; and b) an external unit (6), connected to the electrode, suitable for generating electric current and reading the impedance.

### STATE OF THE ART

SCS is an effective therapy for the treatment of chronic and intractable pain including diabetic neuropathy, failed back surgery syndrome, complex regional pain syndrome, phantom limb pain, ischemic limb pain, refractory unilateral limb pain syndrome, postherpetic neuralgia and acute herpes zoster pain. Another pain condition that is a potential candidate for SCS treatment is Charcot-Marie-Tooth (CMT) disease, which is associated with moderate to severe chronic extremity pain. SCS therapy consists of the electrical stimulation of the spinal cord to 'mask' pain. The gate theory proposed in 1965 by Melzack and Wall provided a theoretical construct to attempt SCS as a clinical treatment for chronic pain. This theory postulates that activation of large diameter, myelinated primary afferent fibers suppresses the response of dorsal horn neurons to input from small, unmyelinated primary afferents. A simple SCS system consists of three different parts. First, electrodes are implanted to deliver stimulation pulses to the tissue (the pulses are delivered to the tissue through poles located on those electrodes). Second, an electrical pulse generator implanted while is connected to the electrodes via wires, and third a remote control to adjust the stimulus parameters such as pulse width and pulse rate.

More than 50,000 stimulators are implanted annually in the world. Almost all of them are placed in the epidural space. The epidural space is the area between the dura mater and the vertebral wall, containing fat and small blood vessels. The epidural space is located just outside the dural sac which surrounds the spinal cord, nerve roots and is filled with cerebrospinal fluid.

The use of stimulators in the epidural space is associated with some problems, their low energy efficiency being the main one. This is because, if the electrode poles are located in the epidural space, a large part of the current generated by the pulse generator does not reach the spinal cord because of the intervening tissues, such as the dura mater, which would be placed between the poles and the spinal cord. The presence of electrode poles inside the intradural space could solve this problem, since the resistance to the passage of current is close to zero since the cerebrospinal fluid behaves like a conductor.

On the other hand, there is a scientific consensus on the idea that it is desirable to minimize the invasiveness of the procedures to be performed, and the field of spinal stimulation is not an exception. In this regard, the surgical approach aimed at delivering current inside the epidural space involves the dissection of skin and tissues, their opening and the insertion of the devices directly in the desired location. Consequently, this approach should be substituted by other less invasive procedures such as a percutaneous approach wherein the electrodes are directed to the epidural space through a needle without requiring an incision or tissue dissection.

Some attempts have been made to conceptualize the possible placement of electrodes in the subdural space in order to maximize its energy performance. However, in all cases, this procedure involved performing a surgical technique: opening of skin and tissues, their dissection, sometimes the removal of bone sheets until reaching the dural sac and, once exposed, dissection of its layers until reaching the subarachnoid space to place the implant. Moreover, there is the risk of cerebrospinal fluid leakage through the surgical dural gap or hematoma. Consequently, it is recommended to avoid the surgical manipulation of a tissue such as dural tissue, which forms a layer about 300 micrometres thick.

So, in summary, there is an unmet medical need of finding more efficient and less invasive SCS techniques to treat chronic and intractable pain. To date, there are no electrodes designed to be inserted percutaneously into the subarachnoid or intradural space. Current electrodes are designed for the stimulation of the spinal cord by means of stimulators placed in the epidural space which give rise to the drawbacks explained above.

The present invention aims to provide a solution to this problem and it is herein provided an innovative and minimally invasive medical device for spinal cord stimulation which provides a high energy efficiency.

### DESCRIPTION OF THE INVENTION

The present invention refers to a medical device for spinal cord stimulation (SCS) which comprises:
a) An electrode (1), consisting essentially of a plurality of poles (2), suitable for being inserted inside the intradural space (3) through the dura mater (4) via needle-puncture (5) of the skin; and
b) An external unit (6), connected to the electrode, suitable for generating electric current and reading the impedance.

Thus, the medical device of the invention comprises an electrode (1) with a crucial technical feature because its reduced diameter makes it suitable for being inserted inside the intradural space (3) through the dura mater (4), via needle-puncture (5) of the skin (percutaneous technique).

The medical device of the invention is associated with some technical advantages:
- It provides a high energy efficiency: Since the electrode is directly placed inside the intradural space, a large amount of the current generated by the pulse generator reach the spinal cord because there are not structures, like the dura mater, interrupting the passage of current. In fact, in the intradural space, the resistance to the passage of current is close to zero since the cerebrospinal fluid behaves like a current conductor. Thus, the implantation of the stimulator inside the intradural space (3), rather than in the epidural space, directly impacts on the energy efficiency of the system. The intradural space (1) is the area inside the dura mater (4) (inside the dural sac) which is filled with cerebrospinal fluid (CSF) surrounding the medulla. Thus, the stimulation inside the intradural space (3) would be more energy efficient since it could be possible to take advantage of the conductivity of the CSF to exert its action, reducing energy consumption and allowing greater tissue penetration. Moreover, electrical current does not need to go through the dura mater (4) to reach the spinal cord, since the electrical current is directly applied in the intradural space (3). By means of the stimulation in the intradural space, the expected energy consumption is hundreds of times lower (as compared with the stimulation in the epidural space) due to the presence of CSF and no other structures that disperse energy.
- Said high energy efficiency enables the physician to use lower current intensities without jeopardizing the efficiency of the treatment. The use of said low intensities gives rise to the possibility of designing an electrode (1) with a reduced diameter.
- At the same time, said reduced diameter of the electrode (1) gives rise to the possibility of designing a minimally invasive medical devise because the electrode can be inserted in the patient via needle-puncture (5) of the skin. This technique is a relatively minimally invasive procedure involving superficial and controlled puncturing of the skin by rolling with miniature fine needles (5), thus avoiding the need of dissecting skin, fat, muscles and minimal amounts of bone from the spine.

So, the first embodiment of the present invention refers to a medical device for spinal cord stimulation which comprises: a) An electrode (1), consisting essentially of a plurality of poles (2), suitable for being inserted inside the intradural space (3) through the dura mater (4) via needle-puncture of the skin (5); and b) External unit (6), connected to the electrode, suitable for generating electric current and reading the impedance.

In a preferred embodiment, the electrode has a diameter ≤ 1.3 mm. In a preferred embodiment, the electrode has a diameter from 0.15mm to 0.9mm. This is the diameter required by the electrode to be suitable for being inserted inside the intradural space (3) through the dura mater (4) via needle-puncture of the skin (5). Thus, it has a diameter suitable for passing through a relatively low-calibre needle (5) to simplify the intrathecal approach, minimize the size of the dural perforation, and avoid CSF leaks. Assuming low-frequency stimulation will be used, the electrode could have a diameter of 0.15 mm, which would allow the use a very fine lead wire (and easier to implant), preferably covered by an insulating layer.

In a preferred embodiment, the diameter of the needle is ≤ 1.31mm, preferably ≤ 0.91 mm.

In a preferred embodiment, the electrode (1) comprises means, for instance a coating or cover (7), that facilitates macrophage adhesion and fibrous reaction, to be fixed to the ligamentous or muscular structures of the extradural space once at least two poles have reached the intradural space. So, it comprises at least two stimulation poles to create the intrathecal electric field. According to this preferred embodiment, once the poles have reached the intradural space (this is indicated by the impedance shown by the external unit), the electrode can be fixed to the ligamentous or muscular structures of the extradural space to avoid the backward movement of the electrode and thereby prevent the poles from falling out of the intradural space. Since an intrathecal subthreshold stimulation would be used, a due to the energy efficiency of the system, it is possible to propose the mere presence of 2 stimulation poles. Currently, the epidural electrodes have 4, 8 or 16 poles separated by a few millimetres from each other due to the need to "fight" against the dispersion of energy creating complicated combinations of stimulation.

In a preferred embodiment, each pole can be programmed to be active or passive. This gives the possibility of selecting the area to be stimulated inside the subarachnoid or intradural space.

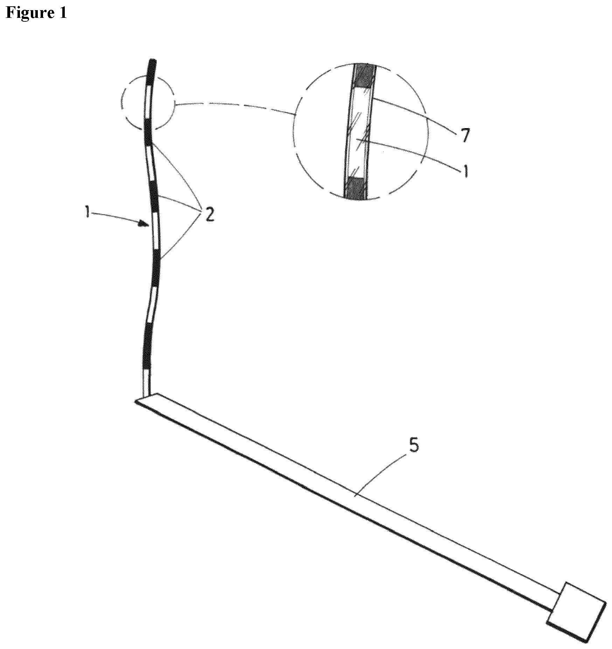

In a preferred embodiment, the electrode is flexible (see **Figure 1**) in order to easily move throughout the intradural space (3) to the desired metameric level for stimulation and avoid damages in the area. Thus, it is flexible and with a minimum of firmness that helps to advance it to the desired metameric level for stimulation.

In a preferred embodiment the tip of the electrode is completely rounded.

In a preferred embodiment, the electrode is covered with a cover (7) that prevents trauma, for example silicone rubber elastomer.

The second embodiment of the present invention refers to a method for treating chronic and intractable pain in a patient by using the medical device of the invention.

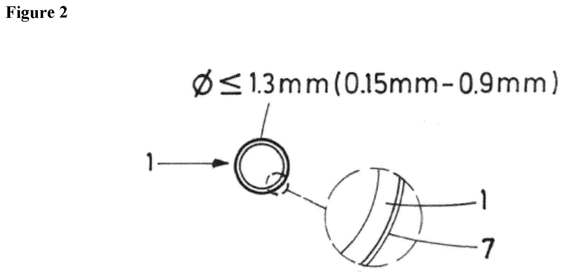

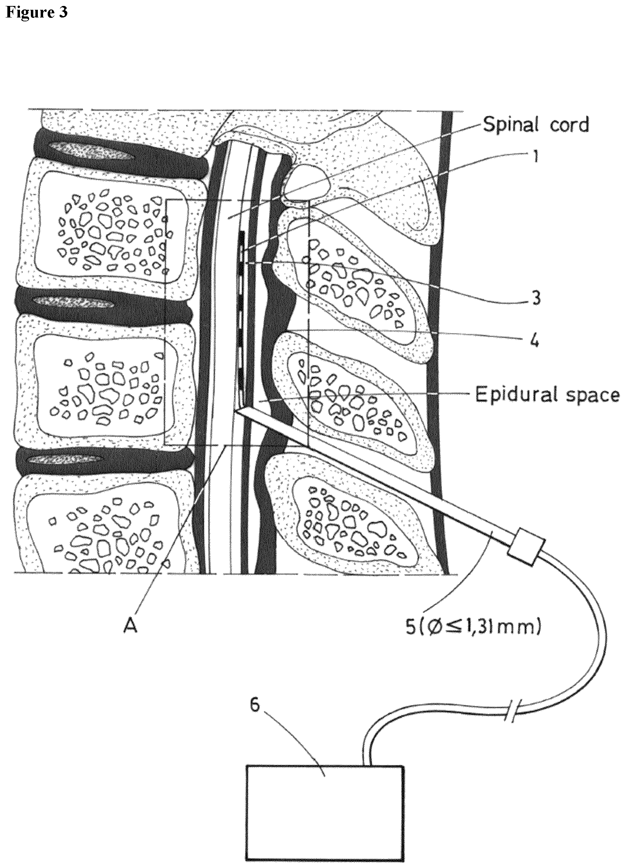

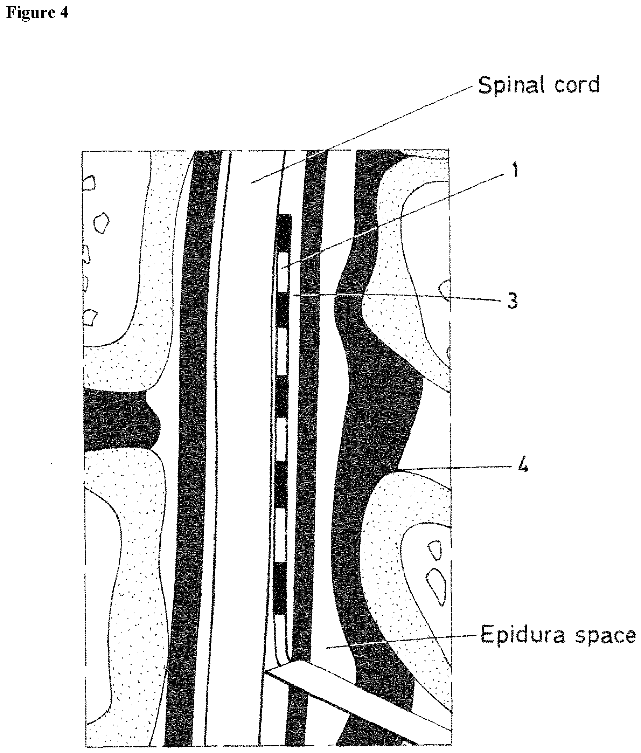

The present invention is better illustrated in **Figures 1** to **4** wherein the following references are cited:

| **Figure reference** | **Technical feature** |
|---|---|
| (1) | electrode |
| (2) | plurality of poles |
| (3) | subarachnoid or intradural space |
| (4) | dura mater |
| (5) | needle |
| (6) | external unit |
| (7) | electrode cover |

### Brief description of the figures

**Figure 1****.** It shows the electrode of the invention (1), which comprises a plurality of poles (2), and a cover (7).
**Figure 2****.** Is shows a sagittal plane illustrating the diameter of the electrode which is ≤ 1.3 mm, preferably between 0.15mm to 0.9mm, in order to be suitable for being inserted inside the intradural space. The electrode cover (7) is also represented in this figure.
**Figure 3**. It shows the electrode of the invention (1) inserted in the intradural space (3), (and not just in the epidural space), through the dura mater (4), by means of a needle (5) which has a dimeter ≤ 1.31mm.
**Figure 4****.** It shows a close view wherein a crucial aspect of the invention is illustrated. The electrode of the invention (1) inserted in the intradural space (3), (and not just in the epidural space), through the dura mater (4).

## Claims

1. A medical device for spinal cord stimulation which comprises:
a. An electrode **(1)** with a diameter ≤ 1.3 mm, consisting essentially of a plurality of poles **(2),** suitable for being inserted inside the intradural space **(3)** through the dura mater **(4)** via needle-puncture **(5)** of the skin;
b. External unit **(6),** connected to the electrode, suitable for generating electric current and reading the impedance.

2. Medical device, according to claim 1, wherein the electrode **(1)** has a diameter from 0.15mm to 0.9mm.

3. Medical device, according any of the previous claims, wherein the electrode **(1)** is flexible in order to easily move throughout the intradural space to the desired metameric level for stimulation and avoid damages in the area.

4. Medical device, according any of the previous claims, wherein the electrode **(1)** is covered with a cover **(7)** that prevents trauma, for example silicone rubber elastomer.

5. Medical device, according to any of the previous claims, wherein the diameter of the needle **(5)** is ≤ 1.31mm, preferably ≤ 0.91 mm.

6. Medical device, according to any of the previous claims, wherein each pole can be programmed to be active or passive.
